# EUROPÄISCHE PATENTSCHRIFT

(11) **EP 2 872 111 B1**
(45) Veröffentlichungstag und Bekanntmachung des Hinweises auf die Patenterteilung: **04.09.2019**
(21) Anmeldenummer: 13737573.9
(22) Anmeldetag: 05.07.2013
(51) Int. Cl.: A61K 8/365, A61K 8/37, A61K 8/81, A61K 8/06, A61Q 19/10, A61K 8/34, A61K 8/39

(54) **VERFAHREN ZUR HERSTELLUNG EINES PFLEGENDEN, KOSMETISCHEN REINIGUNGSMITTELS**
METHOD FOR PRODUCING A CARING COSMETIC CLEANSING COMPOSITIONS
PROCÉDÉ DE FABRICATION D'UNE COMPOSITION NÉTTOYANTE COSMÉTIQUE DE SOIN

(30) Priorität: 11.07.2012 DE 102012212106
(43) Veröffentlichungstag der Anmeldung: 20.05.2015
(73) Patentinhaber: Henkel AG & Co. KGaA, 40589 Düsseldorf (DE)
(72) Erfinder: SARTINGEN, Kirsten, 41379 Brüggen (DE); SCHELGES, Heike, 47877 Willich (DE); HEIDE, Barbara, 47809 Krefeld (DE); GUERRERA, Cecile, F-83000 Toulon (FR)
(86) Internationale Anmeldenummer: PCT/EP2013/064227
(87) Internationale Veröffentlichungsnummer: WO 2014/009263

(56) Entgegenhaltungen:
- EP-A1- 2 327 758
- DE-A1- 10 232 366
- DE-A1-102004 029 328
- US-A1- 2004 234 486

## Beschreibung

Die Erfindung liegt auf dem Gebiet der Kosmetik und betrifft ein Verfahren zur Herstellung eines Reinigungsmittels sowie ein kosmetisches Reinigungsmittel, das neben einem Pigment und/oder einem Trübungs- und/oder Perlglanzmittel mindestens ein Acrylatpolymer, eine O/W-Emulsion sowie eine spezielle Tensidmischung enthält.

Kosmetische Reinigungsmittel sind lange bekannt und werden regelmäßig verbessert bzw. den wechselnden Bedürfnissen der Verbraucher angepasst.
Beispielsweise erwarten Verbraucher von einem modernen Reinigungsmittel nicht nur eine gute Reinigung und Erfrischung, sondern auch pflegende Eigenschaften. Insbesondere sollen Reinigungsmittel nach ihrer Anwendung auf der Haut kein Spannungsgefühl und/oder keine Trockenheit hinterlassen. Die Haut soll sich nach der Reinigung vielmehr weich, glatt und befeuchtet anfühlen.
Zudem spielt auch der optische und haptische Eindruck eines Reinigungsmittels eine zunehmend wichtige Rolle, weshalb die Herstellung von Produkten mit reichhaltiger, cremiger Konsistenz besonders erstrebenswert ist.

Es ist bekannt, kosmetischen Reinigungsmitteln zur Verbesserung der Hautpflege pflegende Öle und/oder Wachse hinzuzufügen.
Die Herstellung solcher pflegender Reinigungsmittel ist jedoch problematisch, denn zum einen bereitet die Stabilisierung höherer Öl- und/oder Wachsmengen (die zur Erzielung eines ausreichenden Pflegeeffektes erstrebenswert sind) oftmals erhebliche Schwierigkeiten, und zum anderen können sich höhere Mengen an Ölen und/oder Wachsen in kosmetischen Reinigungsmitteln nachteilig auf die Schaumeigenschaften - insbesondere die Schaummenge - der Mittel auswirken.
Ein weiteres Problem ist, dass angesichts der üblichen, eher kurzen Verweildauer eines Reinigungsmittels auf der Haut nur geringe Mengen an Pflegekomponenten auf der Haut abgeschieden werden können, bevor ein Großteil der Pflegekomponenten wieder abgespült wird.

Es besteht daher weiterhin der Bedarf nach pflegenden kosmetischen Reinigungsmitteln, die über ein einfaches Herstellungsverfahren zugänglich sind, und die einen Konditionierungsvorteil für die Haut bieten.

Der vorliegenden Erfindung lag die Aufgabe zugrunde, ein unkompliziertes Verfahren zur Herstellung eines pflegenden Reinigungsmittels - insbesondere eines pflegenden Hautreinigungsmittels - bereitzustellen.
Das Reinigungsmittel sollte eine effektive Menge mindestens eines Pflegeöls enthalten, ohne dass die Stabilisierung des Pflegeöls bei der Herstellung und/oder Lagerung des Reinigungsmittels zusätzliche, (energetisch) aufwendige Schritte erforderlich macht.
Die gereinigte Haut sollte sich nach der Anwendung der Mittel sauber, geschmeidig und glatt anfühlen.
Ein weiteres Ziel der Erfindung war es pflegende kosmetische Reinigungsmittel herzustellen, die eine Lotion-ähnliche Optik und Haptik aufweisen.

Ein erster Gegenstand der Erfindung ist ein Verfahren zur Herstellung eines kosmetischen Reinigungsmittels, das die folgenden Schritte aufweist:
a) Bereitstellen einer wässrigen Dispersion, die mindestens ein anorganisches Pigment und/oder mindestens ein Trübungs- und/oder Perlglanzmittel enthält,
b) Bereitstellen einer Zubereitung, die mindestens ein Acrylat-Homo- oder Copolymer enthält,
c) Vermischen der wässrigen Dispersion aus Verfahrensschritt a) mit der Zubereitung aus Verfahrensschritt b),
d) Bereitstellen einer O/W-Emulsion,
e) Vermischen der O/W-Emulsion aus Verfahrensschritt d) mit der Mischung aus Verfahrensschritt c),
f) Bereitstellen eines kosmetisch akzeptablen Trägers, der mindestens ein Tensid enthält,
g) Vermischen des kosmetisch akzeptablen Trägers aus Verfahrensschritt f) mit der Mischung aus Verfahrensschritt e).

Das anorganische Pigment und/oder das Trübungs- und/oder Perlglanzmittel wird (werden) in Verfahrensschritt a) als wässrige Dispersion vorgelegt. Das Mischungsverhältnis von Wasser zu anorganischem Pigment und/oder Trübungs- und/oder Perlglanzmittel liegt dabei bevorzugt im Bereich von 30 : 1 bis 1 : 1, besonders bevorzugt im Bereich von 20 : 1 bis 5 : 1.
Zur Erzielung des optischen Lotioncharakters des Reinigungsmittels ist es bevorzugt, wenn der Gehalt an anorganischem(n) Pigment(en) und/oder Trübungs- und/oder Perlglanzmittel 0,01 bis 5 Gew.-%, bevorzugt 0,025 bis 3 Gew.-% und insbesondere 0,05 bis 2,5 Gew.-% beträgt, wobei sich die Mengenangaben auf das Gesamtgewicht des kosmetischen Reinigungsmittels beziehen.

Unter geeigneten anorganischen Pigmenten werden bevorzugt Pigmente verstanden, die einen Brechungsindex > 1,8, mehr bevorzugt > 1,9 und insbesondere > 2,0 aufweisen.

Beispiele für besonders geeignete anorganische Pigmente sind die sogenannten Weißpigmente wie Titandioxid, Lithopone, Zinkoxid, Zinksulfid und/oder Calciumcarbonat. Insbesondere geeignet ist Titandioxid, das in Verfahrensschritt a) besonders bevorzugt als 10-20%ige Dispersion in Wasser vorgelegt wird.

Unter geeigneten Perlglanz- und/oder Trübungsmitteln sind beispielsweise
- Mono- und/oder Diester des Ethylenglycols, 1,2-Propandiols, Glycerins und/oder eines Polyethylenglycols mit mindestens einer C₈-C₂₄-Carbonsäure, und/oder
- Styrol/Acrylat-Copolymere zu verstehen.

Besonders geeignet sind die unter den INCI-Bezeichnungen bekannten Trübungs- und/oder Perlglanzmittel:
Glycol Distearate, wie beispielsweise das Handelsprodukt Cutina® AGS der Firma Cognis, Glycol Monostearate, wie beispielsweise das Handelsprodukt Cutina® EGMS der Firma Cognis, PEG-3 Distearate, wie beispielsweise das Handelsprodukt Genapol® TS der Firma Clariant, PEG-2 Distearate, wie beispielsweise das Handelsprodukt Kessco® DEGMS der Firma Akzo Nobel, Propylen Glycol Stearate, wie beispielsweise das Handelsprodukt Tegin® P der Firma Goldschmidt und/oder Styrol/Acrylates-Copolymere wie beispielsweise die Handelsprodukte Joncryl® 67 der Firma Johnson Polymers, Suprawal® WS der Firma BASF und/oder Acusol® OP 301 der Firma Rohm & Haas.

Insbesondere geeignet für die Verwendung in dem Herstellungsverfahren sind die unter den INCI-Bezeichnungen bekannten Trübungs- und/oder Perlglanzmittel:
Glycol Distearate, Glycol Monostearate, PEG-3 Distearate und/oder Styrol/Acrylates-Copolymer.

In Verfahrensschritt b) wird zunächst eine Zubereitung bereitgestellt, die mindestens ein Acrylat-Homo- oder Copolymer enthält. Unter "Zubereitung" wird sowohl das jeweilige, reine Polymerpulver verstanden, als auch eine Lösung, Dispersion oder Emulsion des jeweiligen Polymeren - vorzugsweise in Wasser.
Selbstverständlich kann die Lösung, Dispersion oder Emulsion des jeweiligen Polymeren auch weitere übliche Inhaltsstoffe, wie beispielsweise Konservierungsmittel, enthalten.

In einer bevorzugten Ausführungsform wird das Acrylat-Homo- oder Copolymer als wässrige Lösung, Dispersion oder Emulsion bereitgestellt, und in Verfahrensschritt c) mit der wässrigen Dispersion aus Verfahrensschritt a) vermischt.

Die Reihenfolge der Vermischung der Zubereitungen aus den Verfahrensschritten a) und b) in Verfahrensschritt c) kann beliebig erfolgen (a) zu b) oder b) zu a)).
In einer bevorzugten Ausführungsform wird jedoch die wässrige Dispersion a) zu der Zubereitung b) gegeben.

Die Vermischung in Verfahrensschritt c) erfolgt vorzugsweise bei Raumtemperatur, und es resultiert daraus bevorzugt eine viskose, milchige Zubereitung.
In einer weiteren bevorzugten Ausführungsform kann es von Vorteil sein, den pH-Wert der Mischung aus Verfahrensschritt c) auf einen Wert im Bereich von etwa 4 bis 7, bevorzugt auf einen Wert im Bereich von 5-7, und insbesondere auf einen Wert im Bereich von 6-7 einzustellen.

Unter geeigneten Acrylat-Homo- und -Copolymeren sind bevorzugt vernetzte oder unvernetzte Polyacrylate und/oder vernetzte oder unvernetzte Copolymere von (Meth)acrylsäure mit mindestens einem (Meth)acrylsäureester zu verstehen.
Vorzugsweise handelt es sich um anionische Polymere, die gegebenenfalls hydrophob modifiziert sein können.

Beispiele für anionische Monomere, aus denen die Acrylat-Homo- und -Copolymere bestehen können, sind Acrylsäure, Methacrylsäure, Crotonsäure, Itaconsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und (Meth)acrylsäure.

Bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichnen Carbopol® im Handel erhältlich.
Ebenfalls bevorzugt ist das Homopolymer der 2-Acrylamido-2-methylpropansulfonsäure, das beispielsweise unter der Bezeichnung Rheothik®11-80 im Handel erhältlich ist.
Weiterhin bevorzugt sind unvernetzte und vernetzte, hydrophob modifizierte Polyacrylsäuren, die als etwa 30%ige Emulsionen in Wasser, beispielsweise unter den Handelsbezeichnungen Carbopol® Aqua SF1, Carbopol® Aqua SF2 oder Rheomer® 33, von verschiedenen Anbietern erhältlich sind.

Unter bevorzugten anionischen Acrylat-Copolymeren sind Copolymere aus mindestens einem anionischen Monomer und mindestens einem nichtionogenen Monomer zu verstehen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Itaconsäuremono- und -diester, Vinylpyrrolidinon, Vinylether und Vinylester.

Bevorzugte anionische Copolymere sind beispielsweise Copolymere aus Acrylsäure, Methacrylsäure und/oder deren C₁-C₆-Alkylestern, wie sie unter der INCI-Deklaration Acrylates Copolymer vertrieben werden. Bevorzugte Handelsprodukte sind beispielsweise Aculyn® 33 der Firma Rohm & Haas und/oder Rheocare® TTA der Firma Cognis. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20. Derartige Copolymere werden von der Firma Rohm & Haas unter der Handelsbezeichnung Aculyn® 22 sowie von der Firma National Starch unter den Handelsbezeichnungen Structure® 2001 und Structure® 3001 vertrieben.

Es wurde gefunden, dass wässrige Emulsionen oder Dispersionen der zuvor genannten vernetzten oder unvernetzten, hydrophob modifizierten Polyacrylate und/oder vernetzten oder unvernetzten (Meth)acrylsäure-(Meth)acrylsäureester-Copolymere besonders geeignet für die Verwendung in dem erfindungsgemäßen Verfahren sind, denn sie bilden bereits bei Raumtemperatur und pH-Werten im leicht aciden bis neutralen pH-Bereich Gelnetzwerke aus, durch die nicht nur höhere Mengen der O/W-Emulsion über einen langen Zeitraum in dem Reinigungsmittel stabilisiert werden können, sondern durch die gleichzeitig die in dem Reinigungsmittel unlöslichen Pigmente, Perlglanz- und/oder Trübungsmittel stabil suspendiert bleiben.

In Verfahrensschritt d) wird eine O/W-Emulsion bereitgestellt.
Die für das erfindungsgemäße Verfahren geeignete O/W-Emulsionen enthalten üblicherweise mindestens ein Öl, Wasser und mindestens einen Emulgator. Sie werden nach den üblichen, im Stande der Technik bekannten Verfahren hergestellt.
Alternativ kann in Verfahrensschritt d) als geeignete O/W-Emulsion auch eine vorgefertigte, im Handel erhältliche Emulsion bereitgestellt werden.

Die O/W-Emulsion aus Verfahrensschritt d) wird in dem erfindungsgemäßen Verfahren bevorzugt in einer Menge von 0,1 bis 20 Gew.-%, bevorzugt in einer Menge von 0,2 bis 15 Gew.-% und insbesondere in einer Menge von 0,3 bis 10 Gew.-% eingesetzt, wobei sich die Mengenangaben auf das Gesamtgewicht des Reinigungsmittels beziehen.

Geeignete Öle für die Herstellung der O/W-Emulsionen können ausgewählt sein aus mineralischen, natürlichen und synthetischen Ölkomponenten und/oder aus Fettstoffen.

Als natürliche (pflanzliche) Öle können Triglyceride und Mischungen von Triglyceriden eingesetzt werden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter und Shea-Butter.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol® S).
Als synthetische Öle kommen Silikonverbindungen in Betracht.
Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als Ölkomponente kann weiterhin ein Dialkylether dienen.
Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.
Besonders bevorzugt ist der Di-n-octylether, der im Handel unter der Bezeichnung Cetiol® OE erhältlich ist.

Unter Fettstoffen sind zu verstehen Fettsäuren, Fettalkohole sowie natürliche und synthetische Wachse, welche sowohl in fester Form als auch flüssig in wässriger Dispersion vorliegen können. Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
Besonders bevorzugt sind üblicherweise die Fettsäureschnitte, welche aus Cocosöl oder Palmöl erhältlich sind; insbesondere bevorzugt ist in der Regel der Einsatz von Stearinsäure.
Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können auch Wollwachsalkohole, wie sie beispielsweise unter den Bezeichnungen Corona®, White Swan®, Coronet® oder Fluilan® käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere geeignete Fettstoffe sind beispielsweise
- Esteröle. Unter Esterölen sind zu verstehen die Ester von C₆ - C₃₀ - Fettsäuren, insbesondere von C₅ - C₁₈ - Fettsäuren mit C₂ - C₃₀ - Fettalkoholen, insbesondere mit C₁₀ - C₂₂ - Fettalkoholen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Cetyl Palmitate, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Din-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol® CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise die Handelsprodukte Tegin® M, Monomuls® 90-O18, Monomuls® 90-L12, Cetiol® HE oder Cutina® MD.

In einer bevorzugten Ausführungsform enthält die O/W-Emulsion mindestens ein natürliches Öl und/oder mindestens einen der zuvor genannten Fettsäureester und/oder mindestens einen der zuvor genannten Glycerinmono- oder -diester und/oder mindestens einen der zuvor genannten Fettalkohole.

Zur Herstellung geeigneter O/W-Emulsionen werden bevorzugt 0,5 bis 50 Gew.-%, mehr bevorzugt 1 bis 45 und insbesondere 5 bis 40 Gew.-% eines oder mehrerer der zuvor genannten Öle eingesetzt, wobei sich die Mengenangaben auf das Gesamtgewicht der O/W-Emulsion beziehen.

Ein weiterer wesentlicher Inhaltsstoff der O/W-Emulsion in Verfahrensschritt d) ist Wasser. Die O/W-Emulsion enthält - bezogen auf ihr Gesamtgewicht - vorzugsweise 30 bis 90 Gew.-%, mehr bevorzugt 40 bis 80 Gew.-% und insbesondere 45 bis 75 Gew.-% Wasser.

Geeignete Emulgatoren werden in den O/W-Emulsionen - bezogen auf deren Gesamtgewicht - bevorzugt in einer Menge von 0,1 bis 50 Gew.-%, mehr bevorzugt von 0,5 bis 40 Gew.-% und insbesondere von 1 bis 35 Gew.-% eingesetzt, und können bevorzugt ausgewählt sein aus
- C₈-C₃₀-Fettsäuremono- und -diestern von Anlagerungsprodukten von 1 bis 30 Mol Ethylenoxid an Glycerin,
- Anlagerungsprodukten von 2 bis 50 Mol Ethylenoxid und/oder 0 bis 5 Mol Propylenoxid an lineare und verzweigte Fettalkohole mit 8 bis 30 C-Atomen oder an Fettsäuren mit 8 bis 30 C-Atomen und an Alkylphenole mit 8 bis 15 C-Atomen in der Alkylgruppe,
- Sorbitanfettsäureestern und Anlagerungeprodukten von Ethylenoxid an Sorbitanfettsäureester,wie beispielsweise Polysorbate,
- Zuckerfettsäureestern und Anlagerungsprodukten von Ethylenoxid an Zuckerfettsäureester,
- Anlagerungsprodukten von Ethylenoxid an Fettsäurealkanolamide und Fettamine und/oder
- Alkylpolyglucosiden.

In einer bevorzugten Ausführungsform enthalten die O/W-Emulsionen aus Verfahrensschritt d) mindestens einen Emulgator aus der Gruppe der Anlagerungsprodukte von 2 bis 50 Mol Ethylenoxid und/oder 0 bis 5 Mol Propylenoxid an lineare und verzweigte Fettalkohole mit 8 bis 30 C-Atomen. Mehr bevorzugt sind C₁₂-C₂₀-Fettatkohote mit einem Ethoxylierungsgrad von 10 bis 25.

In einer besonders bevorzugten Ausführungsform wird in Verfahrensschritt d) eine O/W-Emulsion bereitgestellt, die neben Wasser
(i) mindestens einen Fettalkohol und/oder mindestens einen ethoxylierten Fettalkohol der nachfolgenden Formel (I), 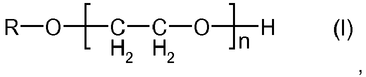 in der R für einen geradkettigen oder verzweigten, gesättigten oder ungesättigten Alkylrest mit 10 bis 22 Kohlenstoffatomen, und der Index n für die Zahl 0 oder eine Zahl von 5 bis 25 steht,
(ii) Glycerin und/oder mindestens einen Glycerinmono- und/oder -diester aus Glycerin und mindestens einer geradkettigen oder verzweigten, gesättigten oder ungesättigten Carbonsäure, die eine C-Kettenlänge von 10 bis 22 Kohlenstoffatomen aufweist,
(iii) mindestens einen Fettsäureester der nachfolgenden Formel (II) enthält 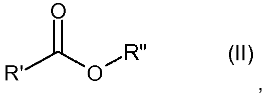 in der R' für einen geradkettigen oder verzweigten, gesättigten oder ungesättigten Alkylrest mit 5 bis 18 Kohlenstoffatomen, und R" für einen geradkettigen oder verzweigten, gesättigten oder ungesättigten Alkylrest mit 10 bis 22 Kohlenstoffatomen steht.

Innerhalb dieser Ausführungsform ist es besonders bevorzugt, wenn die O/W-Emulsion in Verfahrensschritt d)
(i) mindestens einen Fettalkohol und mindestens einen ethoxylierten Fettalkohol gemäß Formel (I),
(ii) Glycerin und mindestens einen Glycerinmono- und/oder-diester, und
(iii) mindestens zwei unterschiedliche Carbonsäureester der Formel (II) enthält.

Ein Beispiel für eine besonders bevorzugte, im Handel erhältliche O/W-Emulsion ist die unter der Bezeichnung Emulgade® CM erhältliche O/W-Emulsion.

In Verfahrensschritt e) wird die O/W-Emulsion aus Verfahrensschritt d) mit der Mischung aus Verfahrensschritt c) vermischt. Die Reihenfolge der Vermischung kann beliebig und vorzugsweise bei Raumtemperatur erfolgen.
Üblicherweise wird jedoch die O/W-Emulsion aus Verfahrensschritt d) unter gelindem Rühren zu der Mischung aus Verfahrensschritt c) gegeben.

In Verfahrensschritt f) wird ein kosmetisch akzeptabler Träger bereitgestellt, der mindestens ein Tensid enthält.

Unter einem kosmetisch akzeptablen Träger wird bevorzugt ein wässriger oder wässrigalkoholischer Träger verstanden.
Bevorzugt enthält der kosmetische Träger mindestens 40 Gew.-% Wasser.
Weiterhin kann der kosmetische Träger 0,01 bis 40 Gew.-%, bevorzugt 0,05 bis 35 Gew.-% und insbesondere 0,1 bis 30 Gew.-% mindestens eines Alkohols enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Sorbitol, Benzylalkohol, Phenoxyethanol oder Mischungen dieser Alkohole.

Bevorzugt sind die wasserlöslichen Alkohole.

Insbesondere bevorzugt sind Ethanol, Ethyldiglykol, 1-Propanol, 2-Propanol, Isopropanol, 1,2-Propylenglycol, Glycerin, Benzylalkohol und/oder Phenoxyethanol sowie Mischungen dieser Alkohole.

Für das erfindungsgemäße Verfahren geeignete Tenside können ausgewählt sein aus gut schäumenden, milden anionischen, amphoteren/zwitterionischen und/oder nichtionischen Tensiden. Zur Erzielung optimaler Milde und zur Vermeidung bzw. Verringerung der Austrocknung der Haut während der Reinigung ist es von Vorteil, wenn in Verfahrensschritt f) eine Mischung aus milden anionischen und milden amphoteren Tensiden in einem zuvor beschriebenen Träger bereitgestellt werden.

Der Gesamttensidgehalt in dem Reinigungsmittel beträgt maximal 12,5 Gew.-% (bezogen auf das Gesamtgewicht des Reinigungsmittels). Geeignete anionische Tenside können in dem erfindungsgemäßen Verfahren bevorzugt in Mengen von 0,1 bis 30 Gew.-%, mehr bevorzugt von 0,5 bis 27,5 Gew.-%, besonders bevorzugt von 1 bis 25 Gew.-% und insbesondere von 3 bis 20 Gew.-% eingesetzt werden, wobei sich die Mengenangaben auf das Gesamtgewicht des Reinigungsmittels beziehen.

Zu den geeigneten anionischen Tensiden zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 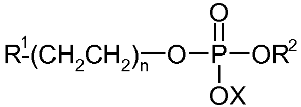 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder - dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten.
Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Geeignete amphotere/zwitterionische Tenside können in dem erfindungsgemäßen Verfahren bevorzugt in Mengen von 0,01 bis 25 Gew.-%, mehr bevorzugt von 0,05 bis 20 Gew.-%, besonders bevorzugt von 0,075 bis 17,5 Gew.-% und insbesondere von 0,1 bis 15 Gew.-% eingesetzt werden, wobei sich die Mengenangaben auf das Gesamtgewicht des Reinigungsmittels beziehen. Geeignete amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,

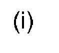

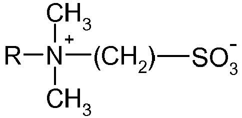

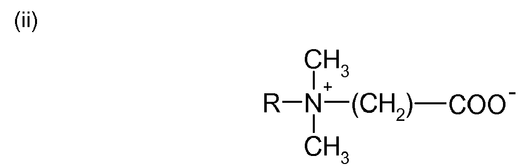

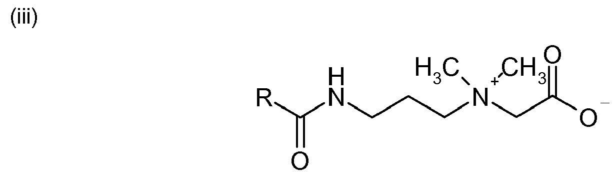

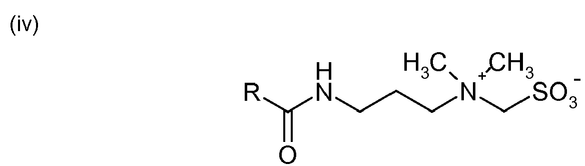

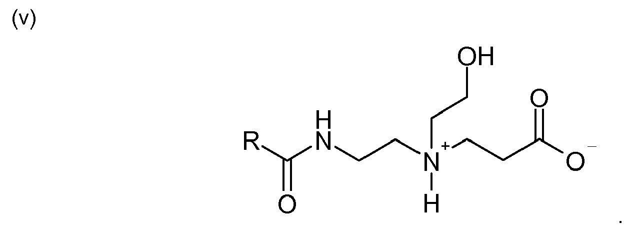

Besonders geeignete amphotere/zwitterionische Tenside sind Alkylamidoalkylbetaine und/oder Alkylampho(di)acetate der zuvor genannten Formeln (i) bis (v).
Zu den insbesondere geeigneten amphoteren/zwitterionischen Tensiden zählen die unter der INCI-Bezeichnung bekannten Tenside Cocamidopropylbetain und Disodium Cocoamphodiacetate.

Geeignete nichtionische Tenside können in dem erfindungsgemäßen Verfahren bevorzugt in Mengen von 0 bis 20 Gew.-%, mehr bevorzugt von 0,25 bis 17,5 Gew.-%, besonders bevorzugt von 0,5 bis 15 Gew.-% und insbesondere von 1 bis 10 Gew.-% eingesetzt werden, wobei sich die Mengenangaben auf das Gesamtgewicht des konditionieren Reinigungsmittels beziehen.

Zu den geeigneten nichtionischen Tensiden/Emulgatoren zählen diejenigen nichtionischen Tenside und Emulgatoren, die bereits an früherer Stelle der Beschreibung genannt wurden.

Für den Fall, dass ein nichtionisches Tensid in dem kosmetischen Träger vorliegt, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Weiterhin bevorzugte nichtionische Tenside, die in dem kosmetischen Träger enthalten sein können, sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

Geeignete Proteinhydrolysate sind vorzugsweise pflanzlichen, tierischen oder marinen Ursprungs und werden in dem erfindungsgemäßen Verfahren bevorzugt in einer Menge von 0,01 bis 10 Gew.-%, mehr bevorzugt von 0,25 bis 7,5 Gew.-% und insbesondere in einer Menge von 0,05 bis 5 Gew.-% eingesetzt, wobei sich die Mengenangaben auf das Gesamtgewicht des konditionierenden Reinigungsmittels beziehen.

Geeignete tierische Proteinhydrolysate sind beispielsweise Elastin-, Kollagen-, Keratin-, Seiden- und/oder Milcheiweiß-Proteinhydrolysate, die auch in Form von Salzen vorliegen können.

Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Geeignete Proteinhydrolysate pflanzlichen Ursprungs sind beispielsweise Soja-, Mandel-, Reis-, Erbsen-, Kartoffel-, Raps- und/oder Weizenproteinhydrolysate.

Solche Produkte sind beispielsweise unter den Warenzeichen Gluadin® (Cognis), DiaMin® (Diamalt), Lexein® (Inolex) und Crotein® (Croda) erhältlich.

Zu den geeigneten Proteinhydrolysaten marinen Ursprunges zählen beispielsweise Kollagenhydrolysate von Fischen oder Algen sowie Proteinhydrolysate von Muscheln bzw. Perlenhydrolysate. Beispiele für geeignete Perlenhydrolysate sind die Handelsprodukte Pearl Protein Extract BG® oder Crodarom® Pearl.

Einsetzbar sind auch kationisierte Proteinhydrolysate, wobei das zugrunde liegende Proteinhydrolysat aus den zuvor beschriebenen tierischen, pflanzlichen und/oder marinen Quellen stammen kann.

Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternärer Ammoniumsalze wie beispielsweise N,N-Dimethyl-N-(n-Alkyl)-N-(2-hydroxy-3-chloro-n-propyl)-ammoniumhalogeniden durchgeführt.
Weiterhin können die kationischen Proteinhydrolysate auch noch weiter derivatisiert sein.
Als typische Beispiele für geeignete kationische Proteinhydrolysate und/oder -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Besonders bevorzugt für die Verwendung in dem erfindungsgemäßen Verfahren sind Proteinhydrolysate aus tierischen Quellen, insbesondere Elastin-, Kollagen-, Keratin- und/oder Seidenproteinhydrolysate, die bevorzugt ein mittleres Molgewicht (Gewichtsmittel) von 100 bis 2500, mehr bevorzugt von 200 bis 2000, besonders bevorzugt von 300 und 1500 und insbesondere von 400 bis 1200 Dalton aufweisen.

Geeignete Handelsprodukte sind beispielsweise von der Firma Croda unter der Bezeichnung ProSina® erhältlich.

Zu den geeigneten nichtionischen Tensiden/Emulgatoren zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine und/oder
- Alkylpolyglucoside.

Für den Fall, dass ein nichtionisches Tensid als weiteres Tensid in dem erfindungsgemäßen Verfahren eingesetzt wird, sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, bevorzugt.

Weiterhin bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fetsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate.

In einer besonders bevorzugten Ausführungsform enthält der kosmetische Träger in Verfahrensschritt f)
a. 3 bis 20 Gew.-% mindestens eines anionischen Tensids und
b. 0,1 bis 15 Gew.-% mindestens eines amphoteren, zwitterionischen oder nichtionischen Tensids,
wobei sich die Mengenangaben auf das Gesamtgewicht des Reinigungsmittels beziehen.

In Verfahrensschritt g) wird schließlich der kosmetische Träger aus Verfahrensschritt f) mit der Zubereitung aus Verfahrensschritt e) vermischt.

Die Vermischung erfolgt vorzugsweise bei Raumtemperatur unter gelindem Rühren.

Es ist dabei sowohl möglich, die Mischung aus Verfahrensschritt e) zu dem Träger aus Verfahrensschritt f) zu geben, als auch den Träger aus Verfahrensschritt f) zu der Mischung aus Verfahrensschritt e).

Gegebenenfalls folgt auf den Verfahrensschritt g) noch ein weiterer Schritt, in dem der pH-Wert und/oder die Viskosität des Reinigungsmittels noch leicht variiert werden können.

Zur Unterstützung der Abscheidung der O/W-Emulsion bzw. der pflegenden Ölkomponente(n) aus der O/W-Emulsion auf der Haut kann es von Vorteil sein, wenn der kosmetische Träger in Verfahrensschritt f) weiterhin mindestens ein kationisches Abscheidungspolymer beinhaltet.

Die Anwesenheit mindestens eines kationischen Polymeren in dem kosmetischen Träger ist zudem bevorzugt, da kationische Polymere auch eine hautkonditionierende Wirkung aufweisen.

Geeignete kationische Polymere können dem kosmetischen Träger bevorzugt in einer Menge von 0,01 bis 20 Gew.-%, mehr bevorzugt in einer Menge von 0,05 bis 15 Gew.-% und insbesondere in einer Menge von 0,1 bis 10 Gew.-% zugesetzt werden.
Das resultierende kosmetische Reinigungsmittel enthält - bezogen auf sein Gesamtgewicht - bevorzugt 0,05 bis 10 Gew.-%, mehr bevorzugt 0,1 bis 5 Gew.-% und insbesondere 0,2 bis 3 Gew.-% mindestens eines kationischen Polymeren.

Geeignete kationische Polymere sind beispielsweise:
- quaternisierte Cellulose-Derivate, wie sie unter den Bezeichnungen Celquat® und Polymer JR® im Handel erhältlich sind,
- hydrophob modifizierte Cellulosederivate, beispielsweise die unter dem Handelsnamen SoftCat® vertriebenen kationischen Polymere,
- kationische Alkylpolyglycoside,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Be zeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
   sowie die unter den Bezeichnungen
- Polyquaternium 2, Polyquaternium 17, Polyquaternium 18, Polyquaternium-24, Polyquaternium 27, Polyquaternium-32, Polyquaternium-37, Polyquaternium 74 und Polyquaternium 89 bekannten Polymere.

Besonders bevorzugte kationische Polymere, die in dem kosmetischen Träger des Verfahrensschrittes f) enthalten sein können, sind quaternisierte Cellulosepolymere, kationische Guarderivate und/oder kationische Polymere auf Acrylsäure(derivat)basis, die insbesondere ausgewählt sind aus den unter den INCI-Bezeichnungen bekannten Polymeren Guar Hydroxypropyltrimonium Chloride, Polyquaternium-6, Polyquaternium-7, Polyquaternium-10, Polyquaternium-37 und/oder Polyquaternium-67.
Insbesondere bevorzugt ist das unter der INCI-Bezeichnung Polyquaternium-7 bekannte kationische Pflege- und Abscheidungspolymer.

Der zuvor beschriebene kosmetische Träger kann noch eine Reihe weiterer fakultativer Wirkstoffe enthalten, die auf der Haut vorteilhafte Eigenschaften bewirken können und das erfindungsgemäße Verfahren nicht erschweren. Zu den bevorzugten fakultativen Wirkstoffen zählen beispielsweise:
- Vitamine, Vitaminderivate und/oder Vitaminvorstufen, die in dem kosmetischen Träger des Verfahrensschritts f) bevorzugt in einer Menge von 0,001 bis 10 Gew.-%, mehr bevorzugt von 0,005 bis 7,5 Gew.-% und insbesondere von 0,01 bis 5 Gew.-% enthalten sein können, und/oder
- Pflanzenextrakte und/oder Pflanzenmilche, die in dem kosmetischen Träger des Verfahrensschritts f) bevorzugt in einer Menge von 0,01 bis 10 Gew.-%, mehr bevorzugt von 0,025 bis 7,5 Gew.-%, besonders bevorzugt von 0,05 bis 5 Gew.-% und insbesondere von 0,075 bis 3 Gew.-% enthalten sein können.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S, 6aR)-2-Oxohexahydrothienol[3,4-d]-imidazol-4-valeriansäure bezeichnet, für die sich aber zwischenzeitlich der Trivialname Biotin durchgesetzt hat.

Bevorzugt ist die Verwendung der Vitamine, Provitamine und Vitaminvorstufen aus den Gruppen A, B, E und H. Insbesondere bevorzugt sind Nicotinsäureamid, Biotin, Pantolacton und/oder Panthenol.

Unter geeigneten Pflanzenextrakten und/oder Pflanzenmilchen sind Extrakte zu verstehen, die aus allen Teilen einer Pflanze hergestellt werden können.
Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.
Besonders bevorzugt für die Verwendung in den erfindungsgemäßen Reinigungsmitteln sind die Extrakte aus Grünem Tee, Brennessel, Hamamelis, Kamille, Aloe Vera, Ginseng, Echinacea purpurea, Olea europea und/oder Boerhavia Diffusa-Wurzeln.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weitere Wirk-, Hilfs- und Zusatzstoffe, die in dem erfindungsgemäßen Verfahren eingesetzt werden können, sind beispielsweise:
- Feuchthaltemittel,
- Parfums,
- UV-Filter,
- Weitere Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Dimethylisosorbid,
- Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Konsistenzgeber wie Zuckerester, Polyolester oder Polyolalkylether,
- Konservierungsmittel, wie beispielsweise Natriumbenzoat und/oder Salicylsäure,
- Viskositätsregler wie Salze (NaCl).

Das erfindungsgemäße Verfahren eignet sich bevorzugt für die Herstellung konditionierender Reinigungsmittel, die bevorzugt einen pH-Wert im Bereich von 3 bis 7, mehr bevorzugt von 4 bis 6,5, besonders bevorzugt von 5 bis 6,25 und insbesondere von 5,5 bis 6 aufweisen.

Die Einstellung der Reinigungsmittel auf den zuvor genannten pH-Bereich weist den Vorteil auf, dass eine zusätzliche Konservierung durch Parabene nicht notwendig ist.

Das erfindungsgemäße Verfahren weist den Vorteil auf, dass es besonders einfach durchzuführen ist und einen geringen Energieaufwand erfordert. Die Zubereitungen der Verfahrensschritte a) und b), c) und d) sowie e) und f) lassen sich in beliebiger Reihenfolge miteinander vermischen, und es können weitere Hilfs- und Wirkstoffe in den kosmetischen Träger eingearbeitet werden, ohne dass das Verfahren dadurch merklich komplizierter wird.

Die resultierenden Reinigungsmittel können einen hohen Anteil an pflegenden Ölkomponenten (in der O/W-Emulsion) enthalten, ohne dass die Stabilität und die Schaumeigenschaften der Mittel negativ beeinträchtigt werden.

Die Mittel hinterlassen auf der Haut nach dem Abspülen ein weiches, geschmeidiges Gefühl.

Ein weiterer Vorteil ist, dass durch das erfindungsgemäße Verfahren kosmetische Reinigungsmittel mit ausgeprägtem Lotioncharakter hergestellt werden können, so dass der Pflegeeffekt Reinigungsmittel visualisiert werden kann.

Ein zweiter Gegenstand der Erfindung ist ein kosmetisches Reinigungsmittel, das
- 0,05 bis 2,5 Gew.-% mindestens eines anorganischen Pigments und/oder mindestens eines Trübungs- und/oder Perlglanzmittels,
- 0,01 bis 10 Gew.-% mindestens eines Acrylat-Homo- oder Copolymeren,
- 0,1 bis 15 Gew.-% mindestens einer O/W-Emulsion, welche
   (i) mindestens einen Fettalkohol und mindestens einen ethoxylierten Fettalkohol der nachfolgenden Formel (I), 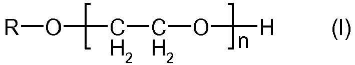 in der R für einen geradkettigen oder verzweigten, gesättigten oder ungesättigten Alkylrest mit 10 bis 22 Kohlenstoffatomen, und der Index n für die Zahl 0 oder eine Zahl von 5 bis 25 steht,
   (ii) Glycerin und mindestens einen Glycerinmono- und/oder -diester aus Glycerin und mindestens einer geradkettigen oder verzweigten, gesättigten oder ungesättigten Carbonsäure, die eine C-Kettenlänge von 10 bis 22 Kohlenstoffatomen aufweist,
   (iii) mindestens zwei unterschiedliche Fettsäureester der nachfolgenden Formel (II) 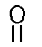 in der R' für einen geradkettigen oder verzweigten, gesättigten oder ungesättigten Alkylrest mit 5 bis 18 Kohlenstoffatomen, und R" für einen geradkettigen oder verzweigten, gesättigten oder ungesättigten Alkylrest mit 10 bis 22 Kohlenstoffatomen steht, enthält
- 3 bis 20 Gew.-% mindestens eines anionischen Tensids, und
- 0,1 bis 15 Gew.-% mindestens eines amphoteren, zwitterionischen oder nichtionischen Tensids enthält, wobei der Gesamttensidgehalt in dem Reinigungsmittel maximal 12,5 Gew.-% beträgt, und wobei sich die Mengenangaben auf das Gesamtgewicht des Reinigungsmittels beziehen. Bezüglich weiterer bevorzugter Ausführungsformen des erfindungsgemäßen Reinigungsmittels gilt mutatis mutandis das zu dem erfindungsgemäßen Verfahren Gesagte.

### Beispiele:

**1.) Duschcreme & Lotion:**

| | |
|---|---|
| Sodium Laureth Sulfate | 9,1 |
| Cocamidopropyl Betaine | 1,9 |
| Acrylates Copolymer | 1,65 |
| PEG-7 Glyceryl Cocoate | 0,8 |
| Perfume | 0,6 |
| Glycerin | 0,51 |
| Sodium Chloride | 0,45 |
| Sodium Benzoate | 0,4 |
| Styrene/Acrylates Copolymer | 0,4 |
| Citric Acid | 0,35 |
| Sodium Hydroxide | 0,34 |
| Polyquaternium-7 | 0,27 |
| Laureth-2 | 0,25 |
| Sodium Salicylate | 0,23 |
| Hydrogenated Castor Oil | 0,1 |
| Propylene Glycol | 0,1 |
| PEG-55 Propylene Glycol Oleate | 0,1 |
| Cetearyl Isononanoate | 0,075 |
| Ceteareth-20 | 0,04 |
| Cetearyl Alcohol | 0,04 |
| Glyceryl Stearate | 0,0125 |
| Panthenol | 0,0075 |
| Ceteareth-12 | 0,005 |
| Cetyl Palmitate | 0,005 |
| Water | ad 100 |

**2.) Perglänzende Duschcreme & Lotion:**

| | |
|---|---|
| Sodium Laureth Sulfate | 9,5 |
| Cocamidopropyl Betaine | 1,9 |
| Acrylates Copolymer | 1,65 |
| PEG-7 Glyceryl Cocoate | 0,8 |
| Perfume | 0,6 |
| Glycerin | 0,5 |
| Sodium Chloride | 0,45 |
| Glycol Distearate | 0,45 |
| Sodium Benzoate | 0,4 |
| Styrene/Acrylates Copolymer | 0,4 |
| Citric Acid | 0,35 |
| Sodium Hydroxide | 0,34 |
| Polyquaternium-7 | 0,27 |
| Laureth-2 | 0,25 |
| Sodium Salicylate | 0,23 |
| Cocamide MEA | 0,225 |
| Hydrogenated Castor Oil | 0,1 |
| Propylene Glycol | 0,1 |
| PEG-55 Propylene Glycol Oleate | 0,1 |
| Laureth-10 | 0,1 |
| Cetearyl Isononanoate | 0,075 |
| Ceteareth-20 | 0,0375 |
| Cetearyl Alcohol | 0,0375 |
| Formic Acid | 0,015 |
| Glyceryl Stearate | 0,0125 |
| Panthenol | 0,0075 |
| Ceteareth-12 | 0,005 |
| Cetyl Palmitate | 0,005 |
| Water | ad 100 |

Die kosmetischen Reinigungsmittel der Beispiele 1 bis 2 wurden nach dem folgenden Verfahren hergestellt:
a) Bereitstellen einer wässrigen Dispersion, die Formic Acid, Laureth-10, Cocamide MEA, Glycol Distearate, Panthenol, Glycerin, Sodium Benzoate, Sodium Salicylate, Polyquaternium-7, Cocamidopropyl Betaine, Cetearyl Isononanoate, Ceteareth-20, Cetearyl Alcohol, Glyceryl Stearate, Ceteareth-12, Cetyl Palmitate und Sodium Laureth Sulfate enthält.
b) Bereitstellen einer Zubereitung, die Acrylates Copolymer, Wasser, NaOH und Sodium Laureth Sulfate enthält,
c) Vermischen der wässrigen Dispersion aus Verfahrensschritt a) mit der Zubereitung aus Verfahrensschritt b),
d) Bereitstellen einer O/W-Emulsion aus Hydrogenated Castor Oil, PEG-7 Glyceryl Cocoate und Wasser,
e) Vermischen der O/W-Emulsion aus Verfahrensschritt d) mit der Mischung aus Verfahrensschritt c),
f) Bereitstellen eines kosmetisch akzeptablen Trägers aus den restlichen Inhaltsstoffen, der u.a. Wasser, Laureth-2, PEG-55 Propylene Glycol Oleate und Propylene Glycol enthält,
g) Vermischen des kosmetisch akzeptablen Trägers aus Verfahrensschritt f) mit der Mischung aus Verfahrensschritt e).

## Patentansprüche

1. Verfahren zur Herstellung eines kosmetischen Reinigungsmittels, das die folgenden Schritte aufweist:
a) Bereitstellen einer wässrigen Dispersion, die mindestens ein anorganisches Pigment und/oder mindestens ein Trübungs- und/oder Perlglanzmittel enthält,
b) Bereitstellen einer Zubereitung, die mindestens ein Acrylat-Homo- oder Copolymer enthält,
c) Vermischen der wässrigen Dispersion aus Verfahrensschritt a) mit der Zubereitung aus Verfahrensschritt b),
d) Bereitstellen einer O/W-Emulsion,
e) Vermischen der O/W-Emulsion aus Verfahrensschritt d) mit der Mischung aus Verfahrensschritt c),
f) Bereitstellen eines kosmetisch akzeptablen Trägers, der mindestens ein Tensid enthält,
g) Vermischen des kosmetisch akzeptablen Trägers aus Verfahrensschritt f) mit der Mischung aus Verfahrensschritt e).

2. Verfahren nach Anspruch 1, **dadurch gekennzeichnet, dass** die wässrige Dispersion in Verfahrensschritt a) 0,01 bis 5 Gew.-%, bevorzugt 0,025 bis 3 Gew.-% und insbesondere 0,05 bis 2,5 Gew.-%
- mindestens eines anorganischen Pigments eines Brechungsindexes > 1,8,
- mindestens eines Mono- und/oder Diesters des Ethylenglycols, 1,2-Propandiols, Glycerins und/oder eines Polyethylenglycols mit mindestens einer C₈-C₂₄-Carbonsäure, und/oder
- mindestens eines Styrol/Acrylat-Copolymeren enthält,
wobei sich die Mengenangaben auf das Gesamtgewicht des kosmetischen Reinigungsmittels beziehen.

3. Verfahren nach einem der Ansprüche 1 oder 2, **dadurch gekennzeichnet, dass** das Acrylat-Homo- oder Copolymer ausgewählt ist aus vernetzten oder unvernetzten, hydrophob modifizierten Polyacrylaten und/oder aus vernetzten oder unvernetzten Copolymeren der (Meth)acrylsäure mit mindestens einem (Meth)acrylsäureester.

4. Verfahren nach einem der Ansprüche 1 bis 3, **dadurch gekennzeichnet, dass** die O/W-Emulsion in Verfahrensschritt d) neben Wasser
(i) mindestens einen Fettalkohol und/oder mindestens einen ethoxylierten Fettalkohol der nachfolgenden Formel (I), 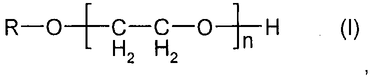 in der R für einen geradkettigen oder verzweigten, gesättigten oder ungesättigten Alkylrest mit 10 bis 22 Kohlenstoffatomen, und der Index n für die Zahl 0 oder eine Zahl von 5 bis 25 steht,
(ii) Glycerin und/oder mindestens einen Glycerinmono- und/oder -diester aus Glycerin und mindestens einer geradkettigen oder verzweigten, gesättigten oder ungesättigten Carbonsäure, die eine C-Kettenlänge von 10 bis 22 Kohlenstoffatomen aufweist,
(iii) mindestens einen Fettsäureester der nachfolgenden Formel (II) 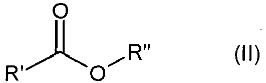 in der R' für einen geradkettigen oder verzweigten, gesättigten oder ungesättigten Alkylrest mit 5 bis 18 Kohlenstoffatomen, und R" für einen geradkettigen oder verzweigten, gesättigten oder ungesättigten Alkylrest mit 10 bis 22 Kohlenstoffatomen steht, enthält.

5. Verfahren nach Anspruch 4, **dadurch gekennzeichnet, dass** die O/W-Emulsion in Verfahrensschritt d)
(i) mindestens einen Fettalkohol und mindestens einen ethoxylierten Fettalkohol gemäß Formel (I),
(ii) Glycerin und mindestens einen Glycerinmono- und/oder-diester, und
(iii) mindestens zwei unterschiedliche Carbonsäureester der Formel (II) enthält.

6. Verfahren nach einem der Ansprüche 1 bis 5, **dadurch kennzeichnet, dass** die O/W-Emulsion in einer Menge von 0,1 bis 20 Gew.-%, bevorzugt in einer Menge von 0,2 bis 15 Gew.-% und insbesondere in einer Menge von 0,3 bis 10 Gew.-% eingesetzt wird, wobei sich die Mengenangaben auf das Gesamtgewicht des Reinigungsmittels beziehen.

7. Verfahren nach einem der Ansprüche 1 bis 6, **dadurch gekennzeichnet, dass** der kosmetische Träger in Verfahrensschritt f)
a. 3 bis 20 Gew.-% mindestens eines anionischen Tensids und
b. 0,1 bis 15 Gew.-% mindestens eines amphoteren, zwitterionischen oder nichtionischen Tensids enthält,
wobei sich die Mengenangaben auf das Gesamtgewicht des Reinigungsmittels beziehen.

8. Verfahren nach einem der Ansprüche 1 bis 7, **dadurch gekennzeichnet, dass** der kosmetische Träger in Verfahrensschritt f) zusätzlich 0,01 bis 20 Gew.-%, bevorzugt 0,05 bis 15 Gew.-% und insbesondere 0,1 bis 10 Gew.-% mindestens eines kationischen Polymeren enthält, wobei sich die Mengenangaben auf das Gesamtgewicht des Reinigungsmittels beziehen.

9. Kosmetisches Reinigungsmittel, enthaltend
- 0,05 bis 2,5 Gew.-% mindestens eines anorganischen Pigments und/oder mindestens eines Trübungs- und/oder Perlglanzmittels,
- 0,01 bis 10 Gew.-% mindestens eines Acrylat-Homo- oder Copolymeren,
- 0,1 bis 15 Gew.-% mindestens einer O/W-Emulsion, welche
(i) mindestens einen Fettalkohol und mindestens einen ethoxylierten Fettalkohol der nachfolgenden Formel (I), 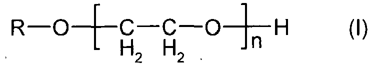 in der R für einen geradkettigen oder verzweigten, gesättigten oder ungesättigten Alkylrest mit 10 bis 22 Kohlenstoffatomen, und der Index n für die Zahl 0 oder eine Zahl von 5 bis 25 steht,
(ii) Glycerin und mindestens einen Glycerinmono- und/oder -diester aus Glycerin und mindestens einer geradkettigen oder verzweigten, gesättigten oder ungesättigten Carbonsäure, die eine C-Kettenlänge von 10 bis 22 Kohlenstoffatomen aufweist,
(iii) mindestens zwei unterschiedliche Fettsäureester der nachfolgenden Formel (II) 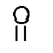 in der R' für einen geradkettigen oder verzweigten, gesättigten oder ungesättigten Alkylrest mit 5 bis 18 Kohlenstoffatomen, und R" für einen geradkettigen oder verzweigten, gesättigten oder ungesättigten Alkylrest mit 10 bis 22 Kohlenstoffatomen steht, enthält
- 3 bis 20 Gew.-% mindestens eines anionischen Tensids, und
- 0,1 bis 15 Gew.-% mindestens eines amphoteren, zwitterionischen oder nichtionischen Tensids,
wobei der Gesamttensidgehalt in dem Reinigungsmittel maximal 12,5 Gew.-% beträgt, und wobei sich die Mengenangaben auf das Gesamtgewicht des Reinigungsmittels beziehen.

## Claims

1. A method for preparing a cosmetic cleansing agent, comprising the following steps:
a) providing an aqueous dispersion which contains at least one inorganic pigment and/or at least one opacifier and/or pearlescing agent,
b) providing a preparation which contains at least one acrylate homopolymer or acrylate copolymer,
c) mixing the aqueous dispersion from method step a) with the preparation from method step b),
d) providing an O/W emulsion,
e) mixing the O/W emulsion from method step d) with the mixture from method step c),
f) providing a cosmetically acceptable carrier which contains at least one surfactant,
g) mixing the cosmetically acceptable carrier from method step f) with the mixture from method step e).

2. The method according to claim 1, **characterized in that** the aqueous dispersion in method step a) contains from 0.01 to 5 wt.%, preferably from 0.025 to 3 wt.%, and in particular from 0.05 to 2.5 wt.%, of
- at least one inorganic pigment having a refractive index > 1.8,
- at least one monoester and/or diester of ethylene glycol, 1,2-propanediol, glycerol and/or a polyethylene glycol with at least one C₈-C₂₄ carboxylic acid, and/or
- at least one styrene/acrylate copolymer,
the amounts stated relating to the total weight of the cosmetic cleansing agent.

3. The method according to one of claims 1 or 2, **characterized in that** the acrylate homopolymer or acrylate copolymer is selected from crosslinked or uncrosslinked hydrophobically modified polyacrylates and/or from crosslinked or uncrosslinked copolymers of (meth)acrylic acid with at least one (meth)acrylic acid ester.

4. The method according to one of claims 1 to 3, **characterized in that** the O/W emulsion in method step d) contains, in addition to water,
(i) at least one fatty alcohol and/or at least one ethoxylated fatty alcohol of the following formula (I), 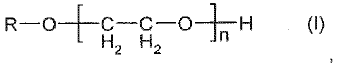 in which R represents a straight-chain or branched, saturated or unsaturated alkyl functional group having 10 to 22 carbon atoms, and the index n represents the number 0 or a number from 5 to 25,
(ii) glycerol and/or at least one glycerol monoester and/or glycerol diester from glycerol and at least one straight-chain or branched, saturated or unsaturated carboxylic acid which has a C chain length of 10 to 22 carbon atoms,
(iii) at least one fatty acid ester of the following formula (II) 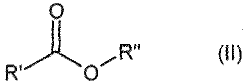 in which R' represents a straight-chain or branched, saturated or unsaturated alkyl functional group having 5 to 18 carbon atoms, and R" represents a straight-chain or branched, saturated or unsaturated alkyl functional group having 10 to 22 carbon atoms.

5. The method according to claim 4, **characterized in that** the O/W emulsion in method step d) contains
(i) at least one fatty alcohol and at least one ethoxylated fatty alcohol according to formula (I),
(ii) glycerol and at least one glycerol monoester and/or glycerol diester, and
(iii) at least two different carboxylic acid esters of formula (II).

6. The method according to one of claims 1 to 5, **characterized in that** the O/W emulsion is used in an amount of from 0.1 to 20 wt.%, preferably in an amount of from 0.2 to 15 wt.%, and in particular in an amount of from 0.3 to 10 wt.%, the amounts stated relating to the total weight of the cleansing agent.

7. The method according to one of claims 1 to 6, **characterized in that** the cosmetic carrier in method step f) contains
a. from 3 to 20 wt.% of at least one anionic surfactant and
b. from 0.1 to 15 wt.% of at least one amphoteric, zwitterionic or non-ionic surfactant, the amounts stated relating to the total weight of the cleansing agent.

8. The method according to one of claims 1 to 7, **characterized in that** the cosmetic carrier in method step f) additionally contains from 0.01 to 20 wt.%, preferably from 0.05 to 15 wt.%, and in particular from 0.1 to 10 wt.%, of at least one cationic polymer, the amounts stated relating to the total weight of the cleansing agent.

9. A cosmetic cleansing agent, containing
- from 0.05 to 2.5 wt.% of at least one inorganic pigment and/or of at least one opacifier and/or pearlescing agent,
- from 0.01 to 10 wt.% of at least one acrylate homopolymer or acrylate copolymer,
- from 0.1 to 15 wt.% of at least one O/W emulsion, which contains
(i) at least one fatty alcohol and at least one ethoxylated fatty alcohol of the following formula (I), 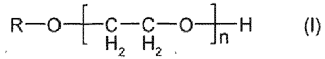 in which R represents a straight-chain or branched, saturated or unsaturated alkyl functional group having 10 to 22 carbon atoms, and the index n represents the number 0 or a number from 5 to 25,
(ii) glycerol and at least one glycerol monoester and/or glycerol diester from glycerol and at least one straight-chain or branched, saturated or unsaturated carboxylic acid which has a C chain length of 10 to 22 carbon atoms,
(iii) at least two different fatty acid esters of the following formula (II) 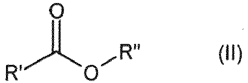 in which R' represents a straight-chain or branched, saturated or unsaturated alkyl functional group having 5 to 18 carbon atoms, and R" represents a straight-chain or branched, saturated or unsaturated alkyl functional group having 10 to 22 carbon atoms,
- from 3 to 20 wt.% of at least one anionic surfactant, and
- from 0.1 to 15 wt.% of at least one amphoteric, zwitterionic or non-ionic surfactant,
wherein the total surfactant content in the cleansing agent is at most 12.5 wt.%, and wherein the amounts stated relate to the total weight of the cleansing agent.

## Revendications

1. Procédé de préparation d'un nettoyant cosmétique comprenant les étapes suivantes :
a) préparer une dispersion aqueuse contenant au moins un pigment inorganique et/ou au moins un agent opacifiant et/ou nacrant,
b) préparer une préparation contenant au moins un homopolymère ou copolymère d'acrylate,
c) mélanger la dispersion aqueuse de l'étape de procédé a) avec la préparation de l'étape de procédé b),
d) préparer une émulsion H/E,
e) mélanger l'émulsion H/E de l'étape de procédé d) avec le mélange de l'étape de procédé c),
f) préparer un véhicule cosmétiquement acceptable contenant au moins un agent tensioactif,
g) mélanger le véhicule cosmétiquement acceptable de l'étape de procédé f) avec le mélange de l'étape de procédé e).

2. Procédé selon la revendication 1, **caractérisé en ce que** la dispersion aqueuse de l'étape de procédé a) contient 0,01 à 5 % en poids, de préférence 0,025 à 3 % en poids et en particulier 0,05 à 2,5 % en poids
- d'au moins un pigment inorganique ayant un indice de réfraction > 1,8,
- d'au moins un mono et/ou diester d'éthylène glycol, de 1,2-propanediol, de glycérol et/ou de polyéthylène glycol avec au moins un acide carboxylique en C₈-C₂₄, et/ou
- d'au moins un copolymère de styrène/acrylate,
les quantités indiquées se rapportant au poids total du nettoyant cosmétique.

3. Procédé selon l'une des revendications 1 ou 2, **caractérisé en ce que** l'homopolymère ou le copolymère d'acrylate est choisi parmi des polyacrylates réticulés ou non réticulés, modifiés hydrophobiquement et/ou parmi des copolymères réticulés ou non réticulés d'acide (méth)acrylique avec au moins un ester d'acide (méth)acrylique.

4. Procédé selon l'une des revendications 1 à 3, **caractérisé en ce que** l'émulsion H/E de l'étape de procédé d) contient, outre de l'eau,
(i) au moins un alcool gras et/ou au moins un alcool gras éthoxylé de formule (I) ci-après, 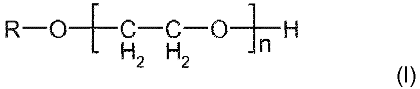 dans laquelle R représente un radical alkyle linéaire ou ramifié, saturé ou insaturé comportant 10 à 22 atomes de carbone, et l'indice n représente le chiffre 0 ou un nombre allant de 5 à 25,
(ii) du glycérol et/ou au moins un mono et/ou diester de glycérol constitué de glycérol et d'au moins un acide carboxylique linéaire ou ramifié, saturé ou insaturé ayant une longueur de chaîne C de 10 à 22 atomes de carbone,
(iii) au moins un ester d'acide gras de formule (II) ci-après 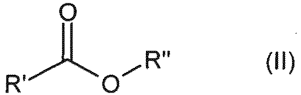 dans laquelle R' représente un radical alkyle linéaire ou ramifié, saturé ou insaturé comportant 5 à 18 atomes de carbone, et R", un radical alkyle linéaire ou ramifié, saturé ou insaturé comportant 10 à 22 atomes de carbone.

5. Procédé selon la revendication 4, **caractérisé en ce que** l'émulsion H/E de l'étape de procédé d) contient
(i) au moins un alcool gras et au moins un alcool gras éthoxylé selon la formule (I),
(ii) du glycérol et au moins un mono et/ou diester de glycérol, et
(iii) au moins deux esters d'acide carboxylique différents de formule (II).

6. Procédé selon l'une des revendications 1 à 5, **caractérisé en ce que** l'émulsion H/E est utilisée en une quantité de 0,1 à 20 % en poids, de préférence en une quantité de 0,2 à 15 % en poids et en particulier en une quantité de 0,3 à 10 % en poids, les quantités indiquées se rapportant au poids total du nettoyant.

7. Procédé selon l'une des revendications 1 à 6, **caractérisé en ce que** le véhicule cosmétique de l'étape de procédé f) contient
a. 3 à 20 % en poids d'au moins un agent tensioactif anionique et
b. 0,1 à 15 % en poids d'au moins un agent tensioactif amphotère, zwitterionique ou non ionique,
les quantités indiquées se rapportant au poids total du nettoyant.

8. Procédé selon l'une des revendications 1 à 7, **caractérisé en ce que** le véhicule cosmétique de l'étape de procédé f) contient, de plus, 0,01 à 20 % en poids, de préférence 0,05 à 15 % en poids et en particulier 0,1 à 10 % en poids d'au moins un polymère cationique, les quantités indiquées se rapportant au poids total du nettoyant.

9. Nettoyant cosmétique, contenant
- 0,05 à 2,5 % en poids d'au moins un pigment inorganique et/ou d'au moins un agent opacifiant et/ou nacrant,
- 0,01 à 10 % en poids d'au moins un homopolymère ou copolymère d'acrylate,
- 0,1 à 15 % en poids d'au moins une émulsion H/E, laquelle contient
(i) au moins un alcool gras et au moins un alcool gras éthoxylé de formule (I) ci-après, 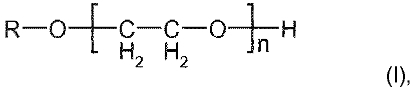 dans laquelle R représente un radical alkyle linéaire ou ramifié, saturé ou insaturé comportant 10 à 22 atomes de carbone, et l'indice n représente le chiffre 0 ou un nombre allant de 5 à 25,
(ii) du glycérol et au moins un mono et/ou diester de glycérol constitué de glycérol et d'au moins un acide carboxylique linéaire ou ramifié, saturé ou insaturé ayant une longueur de chaîne C de 10 à 22 atomes de carbone,
(iii) au moins deux esters d'acide gras différents de formule (II) ci-après 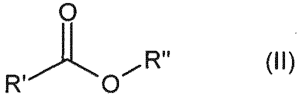 dans laquelle R' représente un radical alkyle linéaire ou ramifié, saturé ou insaturé comportant 5 à 18 atomes de carbone, et R", un radical alkyle linéaire ou ramifié, saturé ou insaturé comportant 10 à 22 atomes de carbone,
- 3 à 20 % en poids d'au moins un agent tensioactif anionique, et
- 0,1 à 15 % en poids d'au moins un agent tensioactif amphotère, zwitterionique ou non ionique,
la teneur totale en agent tensioactif dans le nettoyant étant de 12,5 % en poids maximum, et les quantités indiquées se rapportant au poids total du nettoyant.
